# EUROPEAN PATENT APPLICATION

(11) **EP 1 142 985 A1**
(43) Date of publication of application: **10.10.2001**
(21) Application number: 99959837.8
(22) Date of filing: 16.12.1999
(51) Int. Cl.: C12N 1/12, A23K 1/18

(54) **CHLOROPHYLL-RICH AND SALT-RESISTANT CHLORELLA**

(30) Priority: 17.12.1998 JP 35954998
(71) Applicant: KIRIN BEER KABUSHIKI KAISHA, Tokyo 104-8288 (JP)
(72) Inventor: NAKANISHI;Koichi, Kirin Beer Kabushiki Kaisha, Gunma, 370-1202 (JP)
(74) Representative: Harding, Charles Thomas
(86) International application number: JP9907055
(87) International publication number: WO0036084

(57) **Abstract**

The present invention provides chlorophyll-rich and salt-tolerant chlorella which contains a large amount of chlorophyll, and which makes it possible to supply cultured fishes with little contamination with environmental contaminants such as dioxin or the like having been concentrated through the food-chain from seawater when used as feeds for rotifers for pisciculture, and which can survive a long time in seawater and, consequently, which is suitable for feeds for sea surface pisciculture, fishery feeds and the like. Chlorophyll-rich and salt-tolerant Genus Chlorella mutant strain which can grow in a medium containing 1% by weight or more of sodium chloride and contains chlorophyll at a concentration of 4% or more in total on the basis of dry matter can be constructed by conducting a mutation treatment with a plurality of UV-irradiation and a mutation treatment with a mutagen such as methanesulfonic acid ester, acriflavine or the like.

## Description

### TECHNICAL FIELD

The present invention relates to chlorophyll-rich and salt-tolerant chlorella and the like containing a high concentration of chlorophyll which is a pigment material for food, and has functions of anti-gastric ulcer activity, anti-allergy activity, anti-cerebrovascular disorder activity, reodorizing activity, anti-mutagen activity, dioxin excretion promoting activity and the like; and is excellent as health food, functional food and a material of medicines ; and survives a long time in seawater and, consequently, is suitable for feeds for sea surface pisciculture, fishery feeds and the like.

### BACKGROUND ART

Chlorella is unicellular green algae belong to Chlorococcales, and has been mass cultured as source of health food or as bait for rotifers for pisciculture because it grows relatively speedy and contains a large amount of proteins and essential amino acid lysine and the like. Further, it has been recently reported that chlorophyll containted in chlorella has functions of anti-mutagen activity and dioxin excretion promoting activity besides conventionally reported functions of anti-gastric ulcer activity, anti-hepatopathy activity, anti-allergy activity, anti-cerebrovascular disorder activity, and reodorizing activity ("Food Processing", VOL. 33, NO. 8, 1998, p 18-20). This report says that human takes 90% or more of the total intake amount of dioxin, which is the most problematic environmental contaminants, from food, in particular, 60% of that from fish having been concentrated through the food-chain from the seawater, and that dioxin accumulates in adipose tissue and liver because dioxin generally shows high ratio of absorption in small intestine and is hard to be metabolized.

As techniques for increasing the amount of chlorophyll contained in chlorella, a technique using a culture method wherein a concentration of dissolved oxygen in culture solution is arranged to be in the range of 0.5 to 6 ppm, the culture solution is arranged to be in the range of pH 5.5 to 7.5, hydrolysate of protein or amino acid is added to the solution in the range of 0.1 mg ~ 0.5 g/L-medium, and the amount of glucose in continuous culture solution is arranged to be 10 ~ 40 mg/L-medium, and chlorella is cultured in a tank without irradiation (Japanese Patent Publication No. 58-40462) and a technique using a mutation treatment wherein chlorophyll-rich chlorella that contains chlorophyll at a high concentration of 3% or more in total on the basis of dry matter is constructed by UV-irradiation of chlorella (Japanese Patent Publication No. 2620045) have been conventionally known. However, this mutant strain of chlorophyll-rich genus chlorella that contains chlorophyll at a high concentration of 3% or more in total could not grow in a medium containing sodium chloride.

### DISCLOSURE OF THE INVENTION

Recently, endocrine disrupters have come to draw more and more interest, and food and materials for food which are safe and not contaminated with dioxin or other such environmental contaminants are desired. Fish cultured in seawater and fed with rotifers or the like is not the exception, and there is increasing expectation for fish with little contamination with environmental contaminants such as dioxin or the like having been concentrated through the food-chain from seawater. In order to supply the cultured fishes, it is effective to use chlorophyll-rich chlorella which has anti-mutagen activity and dioxin excretion promoting activity as bait for rotifers or the like, which are baits for the cultured fishes.

In case chlorella is actually used as fishery feeds, chlorella scattered on sea surface will be a live bait for Brachionus plicatilis or the like. However, when chlorella, which has no salt-tolerance, is scattered on sea surface, it is going to die gradually because it cannot grow in seawater, and not only it does not live up to the original expectation as bait for rotifers for piscicultures, but also there is a strong possibility that dead chlorella becomes remaining bait and then contaminates the quality of water. Therefore, conventional chlorella has not been satisfactory in consideration of its utility as fishery feed even though it contains relatively high concentration of chlorophyll. It has not been known how to construct chlorella which is salt-tolerant and adapted for seawater.

An object of the present invention is to provide chlorophyll-rich and salt-tolerant chlorella which contains a large amount of chlorophyll, and which makes it possible to supply cultured fishes with little contamination with environmental contaminants such as dioxin or the like having been concentrated through the food-chain from seawater when used as feeds for rotifers for pisciculture, and which can survive a long time in seawater and, consequently, which is suitable for feeds for sea surface pisciculture, fishery feeds and the like.

The inventors have found that the above object will be attained by constructing chlorella having 2 properties, chlorophyll-richness and salt-tolerance, and have discovered that chlorella which can grow in seawater and contains chlorophyll at a high concentration will be constructed reproducibly by conducting UV-irradiation treatment and acriflavine treatment to chlorella, and the present invention has been thus completed.

The present invention relates to chlorophyll-rich and salt-tolerant chlorella characterized in being able to grow in a medium containing 1% by weight or more of sodium chloride and containing a larger amount of chlorophyll in total than its parent strain, chlorophyll-rich and salt-tolerant chlorella characterized in being able to grow in a medium containing 1% by weight or more of sodium chloride and containing chlorophyll at a concentration of 4% or more in total on the basis of dry matter, and the above-mentioned chlorophyll-rich and salt-tolerant chlorella wherein chlorella is a mutant strain M-207A7 which belongs to Genus Chlorella, salt-tolerant chlorella being able to grow in a medium containing 1% by weight or more of sodium chloride characterized in being obtainable through mutagenization, and chlorophyll-rich chlorella characterized in containing chlorophyll at a concentration of 4% or more in total on the basis of dry matter.

The present invention also relates to a manufacturing method of chlorella algae characterized in cultivating the above-mentioned chlorella by heterotrophic culture method and harvesting chlorophyll-rich and/or salt-tolerant chlorella algae, fishery feeds which have the above-mentioned chlorophyll-rich and salt-tolerant chlorella or salt-tolerant chlorella as active components, a constructing method of a mutant strain characterized in mutagenizing chlorella by UV-irradiation and by methanesulfonic acid ester or acriflavine, and a constructing method of the above-mentioned mutant strain wherein the mutant strain is chlorophyll-rich and salt-tolerant Genus Chlorella mutant strain which can grow in a medium containing 1% by weight or more of sodium chloride and contains chlorophyll at a concentration of 4% or more in total on the basis of dry matter.

Chlorophyll-rich and salt-tolerant chlorella, salt-tolerant chlorella or chlorophyll-rich chlorella of the present invention can be constructed by mutation treatments of a parent chlorella strain. Mutation treatments with UV-irradiation and/or with a mutagen are exemplified as examples of such mutation treatments.

In the present invention, chlorella is defined unicellular green algae which belong to Genus Chlorella of Chlorococcales. As a parent chlorella strain for mutation treatment, for example, wild-type strains separated from water or soil collected from lakes, or preserved strains can be used. Examples of such chlorella which belongs to Genus Chlorella include C. sorokiniana, C. vulgaris, C. ellipsoidea, C. luteoviridis, C. protothecoides, C. zopfingiensis, C. vaiegata, C. xanthella, C. saccharophilia, C. miniata.

A chlorella strain which can be cultured by heterotrophic proliferation wherein organic substance is used as carbon source is preferable for both a parent strain and a mutant strain. As culture medium for the chlorella strain and selective culture medium for the mutant strain, any medium can be used as long as the chlorella strain can proliferate, and examples of such medium are YM broth liquid, YM agar medium whose composition is shown in Table 1 (both of them are made by Difco), and chlorella culture medium whose composition is shown in Table 2.

**Table 1**

| (YM agar medium; made by Difco) | |
|---|---|
| Yeast extract | 3.0 g |
| Malt extract | 5.0 g |
| Bactopeptone | 5.0 g |
| Glucose | 10.0 g |
| Agar | 20.0 g |
| Distilled water | 1000 ml |

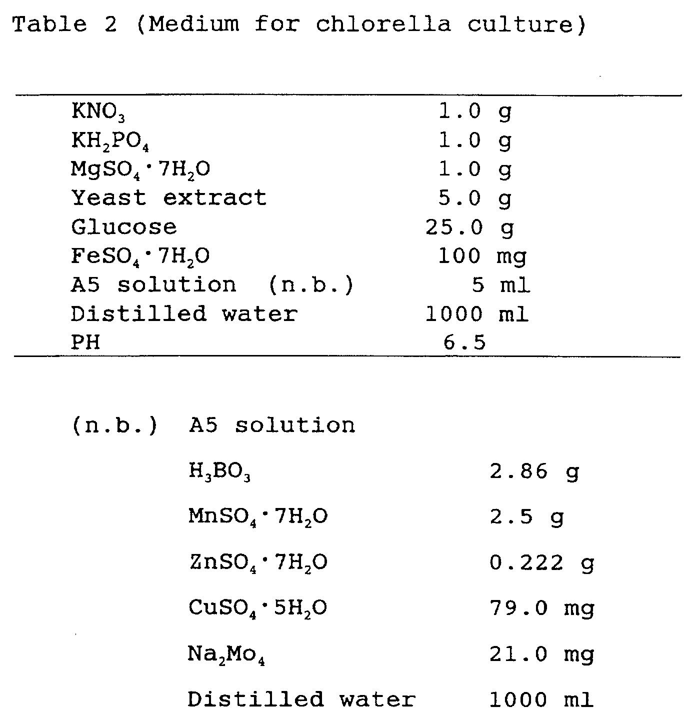

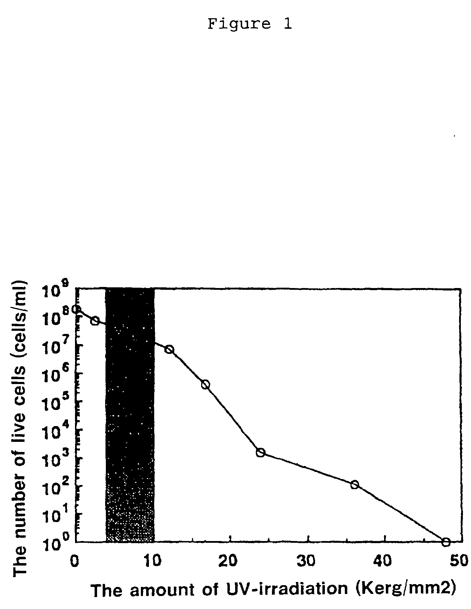

With regard to a mutation treatment with UV-irradiation, any mutation treatment can be used as long as a target mutant strain can be obtained from a parent strain by the treatment. A treatment method using irradiation by UV lamp having wavelength of 254 nm is exemplified as an example of the mutation treatment with UV-irradiation. It is relatively easy, as shown in Fig. 1, to obtain a target mutant strain when the mutation by UV-irradiation is conducted with the UV-irradiation amount in the range of 4 ~ 10 kerg/mm². In many cases, the target strain is more effectively obtainable when irradiation is conducted repeatedly than conducted once.

As a mutation treatment with mutagens, any mutation treatment can be used as long as a target mutant strain can be obtained from a parent strain by the treatment. Examples of the mutation treatment using such mutagens are treatment methods using mutagens such as methanesulfonic acid ester, acriflavine, N-methyl-N'-nitro-N-nitroguanidine, 5-bromouracil, hydroxylamine or the like. Among them, it is preferable to use methanesulfonic acid ester or acriflavine for constructing the target chlorella mutant strain. Though the concentration and time required for treatments with these mutagens are appropriately arranged according to the kind of mutagens to obtain the target strain from the parent strain effectively, usually the treatment is conducted under the condition where the concentration is 1 ~ 500 ppm and time is 10 ~ 60 minutes. For instance, preferable concentrations of methanesulfonic acid ester and acriflavine for the treatment are 20 ~ 200 ppm and 5 ~ 50 ppm respectively.

Further, it is particularly preferable to conduct both mutation treatments with UV-irradiation and with mutagens. For example, it is extremely difficult to construct chlorella of the present invention, such as salt-tolerant chlorella being able to grow in a medium containing 1% by weight or more of sodium chloride, chlorophyll-rich and salt-tolerant chlorella being able to grow in a medium containing 1% by weight or more of sodium chloride and containing a larger amount of chlorophyll in total than its parent strain, particularly chlorophyll-rich and salt-tolerant chlorella being able to grow in a medium containing 1% by weight or more of sodium chloride and containing chlorophyll at a concentration of 4% or more in total on the basis of dry matter, reproducibly with only the mutation treatment with UV-irradiation. However, if both a plurality of UV treatment and mutation treatment with mutagen such as methanesulfonic acid ester, acriflavine or the like are conducted, the above-mentioned chlorella can be constructed reproducibly. According to the inventors, it is confirmed by experiments that the selection ratio of these salt-tolerant strains is about 1/10⁶.

M-207A7 strain, a mutant strain which belongs to Genus Chlorella, was constructed as an example of chlorophyll-rich and salt-tolerant chlorella being able to grow in a medium containing 1% by weight or more of sodium chloride and containing chlorophyll at a concentration of 4% or more in total on the basis of dry matter by conducting combined mutation treatment with UV-irradiation together with a mutation treatment with acriflavine. This M-207A7 strain is preserved in Kirin Beer Kabushiki Kaisha, Applied Bioresearch Center, 3, Miyaharacho, Takasaki-shi, Gunma, and is subdivided under Patent Law Article 27(3). Further, this M-207A7 strain is adopted as CCTCC M 99013 to China Center for Type Culture Collection (Wuhan University, Wuhan 430072 The People's Republic of China) at the date of December 10, 1999.

In the present invention, the expression of "being able to grow in a medium containing 1% by weight or more of sodium chloride" means that chlorella is able to grow when it is inoculated into YM agar medium where 1% by weight of sodium chloride had been added to, and is cultured in a dark place at 35°C for 7 days, and the expression of "containing chlorophyll at a concentration of 4% or more in total on the basis of dry matter" means that chlorella contains chlorophyll at a concentration of 4% or more in total on the basis of dry matter when it is inoculated into a medium for chlorella culture whose composition is shown in the above-mentioned Table 2, and is cultured by shake culture of 200 rpm at 35°C for 3 days.

### BRIEF EXPLANATION OF DRAWING

Fig. 1 is a view showing death rate of chlorella in UV-irradiation.

### BEST MODE FOR CARRYING OUT THE INVENTION

The present invention will be explained in detail with examples, but the technical scope of the present invention is not limited to the examples below.

### Example 1 [Obtaining of chlorophyll-rich and salt-tolerant chlorella]

### (Isolation of a parent strain)

Water and soil were collected from lakes in Gunma prefecture in May to July 1997, and were placed in agar medium where 1% of antibiotic mixed solution N6 (The Society of Fermentation Technology 1991 Conference, Abstract Book p 471) whose composition is shown in Table 4 was added to A10 medium (edited by The University of Tokyo, Institute of Molecular and Cellular Biosciences, IAM Catalogue of Strains. P 404. 1993) shown in Table 3, and cultured at 35°C for 7 days, at 12/12 h light-dark cycle, and unicellular green algae of Genus Chlorella was isolated. Next, the isolated chlorella was completely asepticized by pipette washing method ("Method for Phycological Study", Kyoritsu Shuppan Co., LTD., published on December 1, 1979, p 70). This aseptic strain was inoculated into YM broth liquid and then cultured at 35°C for 7 days in a dark place, and a parent strain was constructed by isolating the proliferated strain with YM agar medium.

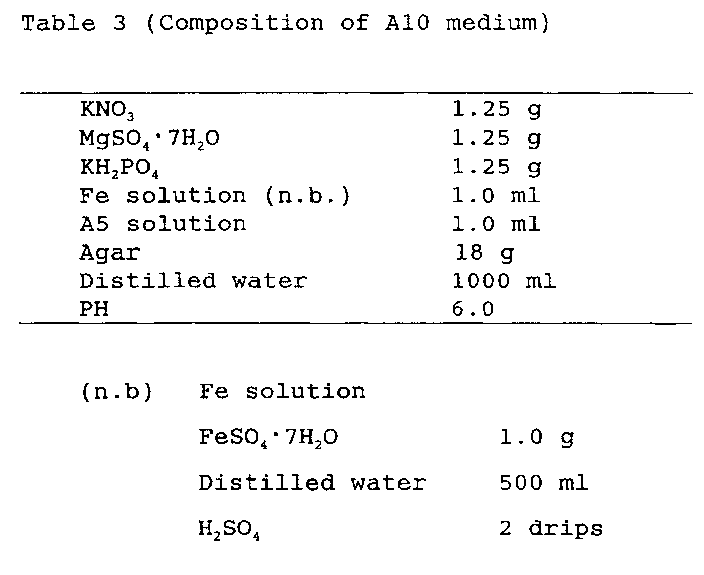

**Table 4**

| (Composition of antibiotic mixed solution N6) | |
|---|---|
| Penicillin G potassium | 334 mg |
| Streptomycin sulfate | 334 mg |
| Kanamycin sulfate | 134 mg |
| Gentamycin sulfate | 134 mg |
| Fosfomycin | 334 mg |
| Nystatin | 26 mg |
| Distilled water | 100 ml |

Used after filtration and sterilization treatment with 0.22 µm membrane filter

### (Selection of chlorophyll-rich mutant strains)

The above-mentioned parent strain was suspended in aseptic water at cell density of 10⁷ ~ 10⁸ cells/ml, and UV-irradiation with irradiation amount of 5 kerg/mm² was conducted twice to the suspension, then the suspension was cultured in YM agar medium at 35°C for 7 days in a dark place. Among colonies emerged, 50 strains of colonies showing green darker than the parent strain were selected and isolated. This isolated 50 mutant strains further had a contact treatment for 30 minutes with 20 ppm of acriflavine, and isolation of darker green colonies was repeated, then 50 strains were selected and isolated again.

### (Selection of salt-tolerant strains)

50 chlorophyll-rich strains were inoculated into YM agar medium where 1% by weight or more of sodium chloride had been added to, and cultured at 35°C for 7 days in a dark place. From 30 colonies emerged, M-207A7 strain, which was chlorophyll-rich and salt-tolerant mutant strain, was selected and isolated. Main characters of this mutant strain are shown in Table 5. The characters indicate that the mutant strain M-207A7 is able to grow in a medium containing 1% by weight of sodium chloride.

**Table 5**

| (Characters and qualities) | |
|---|---|
| Size | 4 ∼ 7 µm |
| Shape | spherical |
| Glucose methylotrophy (5%) | +++ |
| Glucose methylotrophy (10%) | ++ |
| Growth in 1% conc. of sodium chloride | ++ |
| Galactose methylotrophy (1%) | - |
| Ethanol methylotrophy | - |
| Soluble starch | - |

### (Measurement of contained chlorophyll)

Thus obtained M-207A7 strain and B-1 strain available on the market as comparison were cultured in a medium for chlorella culture whose composition is shown in Table 2. Further, Chlorella vulgaris C-27 as control was cultured in A10 medium whose composition is shown in Table 3. Cultivations were conducted as follows; 100 ml of each medium was placed in 500 ml conical flask, and cultivated by shake culture with 200 rpm at 35°C for 3 days, after cultivation, culture solution was centrifuged with 3000 rpm for 15 minutes, and algae were retrieved. The amount of contained chlorophyll in total was measured according to a method in which absorbance at 650 nm and 665 nm of methanol extract liquid of the algae was measured (a method described in p 498 of "Method for Phycological Study", Kyoritsu Shuppan Co. , LTD., published on December 1, 1979). The results are shown in Table 6.

**Table 6**

| Strain | amount of algae(g/l) | amount of chlorophyll(%) | medium |
|---|---|---|---|
| M-207A7 (of this invention) | 6.88 | 4.1 | chlorella culture medium (Table 2) |
| B-1 (goods on the market) | 6.60 | 3.1 | chlorella culture medium (Table 2) |
| C. vulgaris C-27 | 2.62 | 1.7 | A10 medium Composition (Table 3) |

### (Feeding test using rotifers)

3 L of breeding solution for rotifers comprising natural seawater and tap water with the ratio of 3:1 was injected into a 5 L beaker, and rotifers were put in the beaker such that the initial concentration would be 150 ~ 200 individuals/ml, and water temperature was kept at 27°C. 60 v/v% suspension of chlorella PCV (Packed Cell Volume) was fed to rotifers for 4 days as follows; 0.1 ml per 100 rotifers in the morning, and 0.2 ml per 100 rotifers in the evening. This experiment was conducted 7 times, and difference in proliferation of rotifers (4days later) between M-207A7 of this invention and B-1 strain available on the market as comparison was examined. The average results of 7 experiments are shown in Table 7. Table 7 verifies that rotifers eat a large amount of salt-tolerant strain which can survive in seawater.

**Table 7**

| Strain | individuals/ml | proliferation ratio of rotifers |
|---|---|---|
| M-207A7 (of this invention) | 1850 | proliferated by 9 ~ 12 times |
| B-1 (goods on the market) | 1600 | proliferated by 8 ~ 10 times |

### (Mass culture of M-207A7 strain)

40 L of medium described in Table 4 was placed in 50 L jar fermenter, and ventilated at 30 L/minute, stirred at 300 rpm, then cultured in a dark place at 35°C for 3 days. Subsequently the culture solution was collected, centrifuged at 3000 rpm for 15 minutes, and chlorella algae were obtained. The amount of contained chlorophyll in total was calculated by the above method, and was 4.3% (on the basis of dry matter). As a result, it was found that chlorophyll-rich and salt-tolerant chlorella could be mass cultured in industrial scale while keeping its excellent characters, by normal heterotrophic culture method in a tank.

### Example 2 [Reproducibility test]

Chlorophyll-rich and salt-tolerant chlorella was isolated in a same manner as example 1 except that the places of lakes where parent strains were collected were different. As shown in Table 8, chlorophyll-rich and salt-tolerant chlorella which can grow in a medium containing 1% by weight or more of sodium chloride and contains chlorophyll at a concentration of 4% or more in total on the basis of dry matter was obtainable from 8 lakes other than the lake in example 1.

**Table 8**

| Lake | Growth in 1% conc. of sodium chloride | Amount of chlorophyll(%) |
|---|---|---|
| A | ++ | 4.0 |
| B | ++ | 4.1 |
| C | + | 4.2 |
| D | + | 4.1 |
| E | +++ | 4.1 |
| F | ++ | 4.0 |
| G | + | 4.2 |
| H | + | 4.1 |

### Industrial Applicability

Chlorophyll-rich and salt-tolerant chlorella which contains a large amount of chlorophyll, and which makes it possible to supply cultured fishes with little contamination with environmental contaminants such as dioxin or the like having been concentrated through the food-chain from seawater when used as feeds for rotifers for pisciculture, and which can survive a long time in seawater and, consequently, which is suitable for feeds for sea surface pisciculture, fishery feeds and the like can be mass cultured in industrial scale while keeping its excellent characters, by normal heterotrophic culture method in a tank by using the present invention.

## Claims

1. Chlorophyll-rich and salt-tolerant chlorella **characterized in** being able to grow in a medium containing 1% by weight or more of sodium chloride and containing a larger amount of chlorophyll in total than its parent strain.

2. Chlorophyll-rich and salt-tolerant chlorella **characterized in** being able to grow in a medium containing 1% by weight or more of sodium chloride and containing chlorophyll at a concentration of 4% or more in total on the basis of dry matter.

3. Chlorophyll-rich and salt-tolerant chlorella according to claim 2, wherein chlorella is a mutant strain M-207A7 which belongs to Genus Chlorella.

4. Salt-tolerant chlorella being able to grow in a medium containing 1% by weight or more of sodium chloride **characterized in** being obtainable through mutagenization.

5. Chlorophyll-rich chlorella **characterized in** containing chlorophyll at a concentration of 4% or more in total on the basis of dry matter.

6. A manufacturing method of chlorella algae **characterized in** cultivating chlorella according to one of claims 1 to 5 by heterotrophic culture method and harvesting chlorophyll-rich and/or salt-tolerant chlorella algae.

7. Fishery feed **characterized in** having chlorophyll-rich and salt-tolerant chlorella according to one of claims 1 to 3, or salt-tolerant chlorella being able to grow in a medium containing 1% by weight or more of sodium chloride as active components.

8. A constructing method of a mutant strain **characterized in** mutagenizing chlorella by UV-irradiation and by methanesulfonic acid ester or acriflavine.

9. The constructing method of a mutant strain according to claim 8, wherein the mutant strain is chlorophyll-rich and salt-tolerant Genus Chlorella mutant strain which can grow in a medium containing 1% by weight or more of sodium chloride and contains chlorophyll at a concentration of 4% or more in total on the basis of dry matter.
